Europäisches Patentamt
European Patent Office
Office européen des brevets

(19)

(11) Publication number: 0 295 090 A2

(12) EUROPEAN PATENT APPLICATION

(21) Application number: 88305250.8

(22) Date of filing: 09.06.88

(51) Int. Cl.[4]: C 07 H 19/06
// C07H13/04, C07H13/08

(30) Priority: 10.06.87 GB 8713579
10.07.87 GB 8716233

(43) Date of publication of application:
14.12.88 Bulletin 88/50

(84) Designated Contracting States:
AT BE CH DE ES FR GB GR IT LI LU NL SE

(71) Applicant: THE WELLCOME FOUNDATION LIMITED
183-193 Euston Road
London NW1 2BP (GB)

(72) Inventor: Wilson, Jeffrey Douglas
27 Gorham Place
Durham North Carolina 27705 (US)

Almond, Merrick Richard
1214 Chimney Hill Drive
Apex North Carolina 27502 (US)

Rideout, Janet Litster
3101 Morningside Drive
Raleigh North Carolina 27607 (US)

(74) Representative: Garrett, Michael et al
The Wellcome Research Laboratories Group Patents and Agreements Langley Court
Beckenham Kent BR3 3BS (GB)

(54) **Chemical process.**

(57) This invention relates to a new synthetic process for the manufacture of zidovudine from the starting material D-xylose involving:

i) Conversion of D-xylose to a 1-(β-D-xylofuranosyl)thymine derivative;
ii) 2′-Deoxygenation of the thymine derivative; and
iii) 3′-Azidation of the 2′-deoxy compound.

EP 0 295 090 A2

**Description**

## CHEMICAL PROCESS

The present invention relates to a novel process for the preparation of 3′-azido-3′-deoxythmidine and to intermediates of use in such a process.

The preparation of 3′-azido-3′-deoxythymidine was first described by J.P. Horwitz et al., J. Org. Chem., 29, 2076-2078, 1964, and later for example by R.P. Glinski et al., J. Org. Chem, 38, 4299-4305, 1973 and T-S Lin et al., J. Med. Chem., 21 1 109-112 1978. More recently, 3′-azido-3′-deoxythymidine has been discovered to have a potent antiviral activity against the human immunodeficiency virus (HIV), and has been reported to be of potential value for the therapeutic treatment of acquired immune deficiency syndrome (AIDS) (R. Yarchoan et al., The Lancet, 1 (8481), 575-580, Mar 15, 1986). Following further extensive clinical investigation this compound has been found to be of therapeutic benefit in the treatment of AIDS and AIDS-related complex (ARC). 3′-Azido-3′- deoxythymidine (otherwise named 1-(3′-azido-2′-3′-dideoxy-D-erythro- pentofuranosyl)-thymine) has recently been given the approved name zidovudine.

In view of the demand for relatively large quantities of zidovudine to fulfil clinical testing requirements and to meet other demands for the compound, considerable effort has been devoted towards ensuring adequate supplies of the compound on an industrial scale. A major difficulty that has been encountered in the commercial manufacture of zidovudine is that previously reported methods for the preparation of zidovudine (on a laboratory scale) have involved the use of thymidine as a starting material or intermediate. However, thymidine is a relatively expensive material and its commercial availability is relatively limited, which consequently limits the supply of zidovudine when prepared by previously reported methods.

We have therefore investigated alternative routes of synthesis for zidovudine starting from other, less expensive and more readily available, starting materials. As a result of considerable research and development, we have now discovered a synthetic route for zidovudine that uses, as a starting material, D-xylose which is a relatively inexpensive and readily commercially available starting material, thus avoiding the use of thymidine.

The new synthetic route is characterised by a sequence of key steps involving certain novel intermediates for example novel 5′-methoxy carbonyl-protected derivatives described in more detail below. These intermediates may then be converted to zidovudine by a series of steps essentially involving 2′-deoxygenation (which may be photolytic in nature); introduction of a leaving group at the 3′-position of the sugar residue and replacement of said leaving group by an azido group.

The overall synthetic route will be described in relation to the key steps referred to above which essentially involve (i) conversion of D-xylose to a 1-(β-D-xylofuranosyl)thymine derivative, (ii) 2′-deoxygenation of the thymine derivative; and (iii) 3′-azidation and 5′-deprotection of the 2′-deoxy compound.

Thus, the key steps, each of which represents a feature of the present invention, comprise:-

<u>(i) Conversion of D-xylose to 1-(β-D-xylofuranosyl)thymine derivative</u>

a) treating D-xylose, i.e.

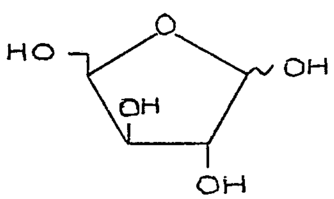

(I)

with a selective hydroxy blocking agent to form a compound of formula (II)

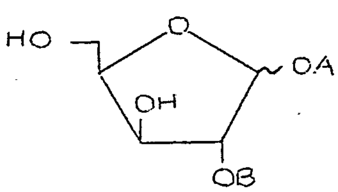

(II)

in which A and B each represent a hydroxy blocking group or together form a single such group serving to block the 1- and 2- hydroxy groups;

b) treating a compound of formula (II) with further hydroxy blocking agents which either selectively

block the 5- and/or 3- hydroxy groups or together form a single group blocking the 5- and 3- hydroxy groups;

i) optionally treating the resulting compound with an agent capable of deblocking the 1- and 2-hydroxy groups and optionally introducing a cyclic sulphite group blocking the 1- and 2-hydroxy groups; or alternatively

ii) treating the resulting compound with an agent serving to introduce a leaving group at the 3-position of the sugar ring and optionally replacing any combined groups blocking the 1- and 2-hydroxy groups with single such blocking groups or a single group blocking the 1- hydroxy group, the 2- hydroxy group being optionally blocked by a photolytically reduceable group; and then

iii) optionally introducing a leaving group at the 1- hydroxy position of the sugar ring.

c) reacting the resulting compound with a thymine derivative of formula (III)

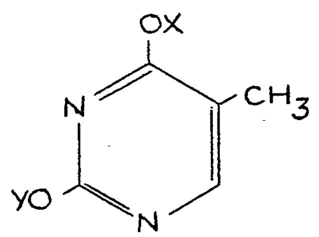

(III)

(wherein X and Y each represent an activating group), to form a compound of formula (IV)

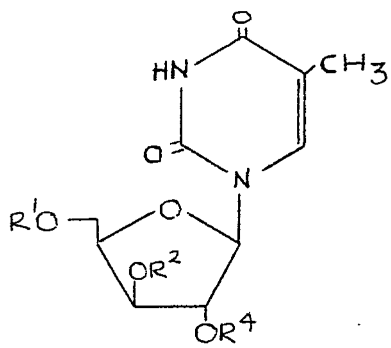

(IV)

(wherein $R^1$ is a hydroxy blocking group, $R^2$ is a leaving group, or $R^1$ and $R^2$ are individual hydroxy blocking groups or together form a single hydroxy blocking group and $R^4$ is hydrogen or a hydroxy blocking group preferably a photolytically reduceable group).

ii) 2′-deoxygenation of the thymine derivative

d) i) optionally deblocking the 3′ and 5′- hydroxy groups and reblocking with alternative blocking groups (wherein $R^4$ is a non-photolytically reduceable hydroxy blocking group, treating a compound of formula (IV) with an agent to remove the 2′- hydroxy blocking group), then treating the 2′- hydroxy compound with an agent serving to provide a photolytically or non-photolytically reduceable group at the 2′- position of the sugar moiety directly, or by :

1) introducing a leaving group at the 2′- position of the sugar moiety;

2) cyclising the compound to form a 2′ ,2- anhydro derivative and either

a) opening the ring to give a compound of formula (V) a or (V)b;

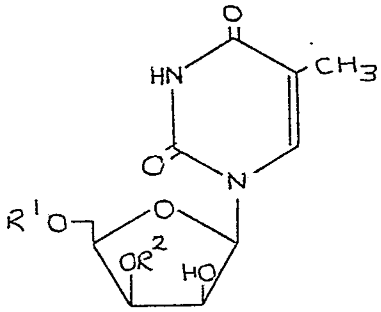

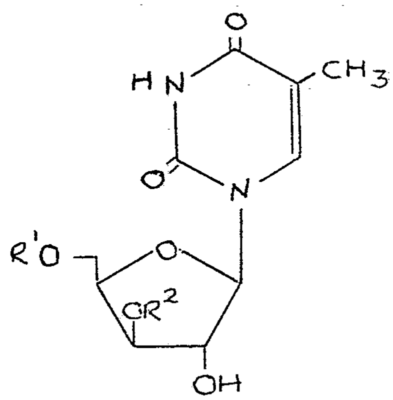

(wherein $R^1$ and $R^2$ are as hereinbefore defined) and introducing a photolytically reduceable group at the 2′- position of the sugar ring; or

b) introducing a non-photolytically reduceable group at the 2′-position of the sugar ring, and reducing to form a compound of formula (VI) below.

d) ii) treating a compound of formula (IV) wherein $R^4$ is a photolytically reduceable group or a compound from d) i) above to deoxygenate the 2′- position sugar moiety to form a compound of formula (VI);

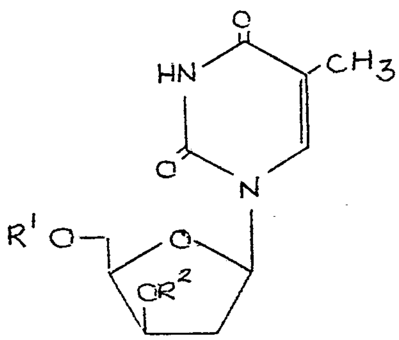

(VI)

(wherein $R^1$ and $R^2$ are as hereinbefore defined)

(e) where a compound of formula (VI) is formed wherein $R^1$ and $R^2$ together form a single hydroxy blocking group, treating the compound with a suitable agent to effect deblocking and selective blocking of the 5′-hydroxy group and introduction of a suitable leaving group at the 3′-position of the 5′- blocked compound.

<u>(iii) 3′-Azidation of the 2′-deoxygenated 1-(β-D-xylofuranosyl)thymine derivative</u>

f) reacting the compound of formula (VI) with an agent serving to introduce an azido group at the 3′-position in the <u>erythro</u> configuration to form a compound of formula (VII)

(VII)

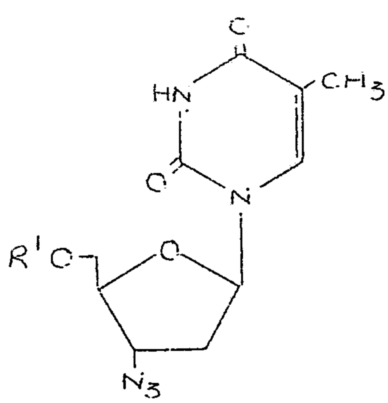

(in which $R^1$ is as hereinbefore defined) and

g) removing the 5′-hydroxy protecting group from the compound of formula (VII) to form zidovudine.

The above procedures (as the preparation of zidovudine from D-xylose are hereinafter described in more detail by way of two alternatives A and B.

Alternative A

With regard to stage a), the 1- and 2-hydroxy groups of xylose may be protected as a ketal (e.g. acetonide) blocking group, for example by treatment of D-xylose with an appropriate agent such as acetone.

With regard to stage b), a single 5′- blocking group may be an alkoxycarbonyl group e.g. methoxycarbonyl, the compound of formula (II) being advantageously reacted with an appropriate chloride, e.g. methoxy -carbonyl chloride, preferably in the presence of a base such as pyridine.

With regard to stage b) option ii) the leaving group may be an organosulphonyloxy (e.g. methanesulphonyloxy, trifluoromethane sulphonyloxy or p-toluenesulphonyloxy) group, the compound of formula (II) being advantageously reacted with an appropriate chloride or anhydride, e.g. methanesulphonyl chloride, preferably in the presenc of a base such as triethylamine, and conveniently in an organic solvent such as dichloromethane. The 1- and 2- hydroxy protecting groups are preferably acyl protecting groups, particularly acetyl or benzoyl protecting groups, the compound of formula (II) being reacted with an appropriate acylating agent such as acetic anhydride in acetic acid, for example in the presence of a mineral acid such as sulphuric acid. The preparation of an analogous compound is also described in J. Am. Chem. Soc.,1958; 80, 5247.

A photolytic reduceable group which is advantageously a benzoate group or substituted benzoate group (e.g. 4-chlorobenzoate or 3-trifluoromethyl benzoate) may be introduced at the 2-hydroxy position of the sugar moiety containing a single group blocking the 1-hydroxy by treatment with an appropriate acid halide (e.g. chloride) or anhydride at ambient temperature in the presence of an aprotic base such as pyridine.

With regard to stage b) iii) the leaving group may be an acyl group particularly acetyl and may be introduced at the 1-hydroxy position of the sugar ring by treatment with an acylating agent such as acetic anhydride in acetic acid.

With regard to stage c) the resulting compound is reacted with a compound of formula (III) in which X and Y are advantageously trialkylsilyl (e.g. trimethylsilyl) groups, the reaction being effected in the presence of a Lewis Acid (e.g. stannic chloride or trimethylsilyl trifluoromethanesulphonate), preferably in an organic solvent such as methylene dichloride or acetonitrile.

With regard to stage d) option i) wherein $R^4$ of compound of formula (IV) is a non-photochemical hydroxy-protecting group, the removal of the 2′-hydroxy blocking group is advantageously effected by treatment of the compound of the formula (IV) under acidic conditions, for example using anhydrous methanolic hydrochloric acid. The 2′-reduceable functional group may be introduced directly as a halo radical in particular a bromo radical using a dialkyl azodicarboxylate, a tri-alkyl or tri-aryl phosphine (eg. $Ph_3P$) in a aprotic solvent (eg. DMF) and a brominating agent eg. an alkaline earth metal bromide such as lithium bromide (LiBr).

Alternatively, the reduceable group may be introduced by the sub-stages i) to iii) in stage d) as follows :

1) The leaving group is advantageously an organosulphonyloxy group (e.g. a methanesulphonyloxy, trifluoromethanesulphonyloxy or p-toluenesulphonyloxy) group, the compound being treated with an appropriate sulphonyl halide (e.g. chloride), as described for stage c).

2) Cyclisation of the compound is conveniently effected by treatment with a base such as potassium carbonate, for example in a solvent such as acetonitrile, preferably at an elevated temperature e.g. reflux temperature.

2 option b)

The reduceable functional group is preferably a halo radical, for example a chloro or iodo radical, but preferably a bromo radical, in which case, the compound is reacted with a brominating agent such as lithium bromide. Alternative reduceable groups include those of formula -CS.R (in which R represents an imidazolyl, $C_{1-4}$ alkoxy (e.g. methoxy), aryloxy (e.g. phenoxy) or $C_{1-4}$ alkylthio group. The reduction of the compound is preferably effected using a reducing agent such as tributyltin hydride, particularly in the presence of azobisisobutyronitrile as a catalyst, preferably in an organic solvent such as toluene.

With regard to stage d) option ii) wherein $R^4$ of a compound of formula (IV) is a photochemical group, 2′-deoxygenation is achieved by photolytic removal of the photochemical group carried out by irradiating the compound of formula (IV) with a high pressure mercury lamp having a pyrex or uranium filter, in the presence of an electron transfer agent such as N-methylcarbazole or N-ethylcarbazole in an aqueous/organic solvent system such as water/THF (1:10) or water/isopropanol (1:10) at about room temperature under an inert atmosphere such as nitrogen. The process is described in J.Amer.Chem.Soc. 1986 Vol 108 Page 3115.

In stage f), the azido group is introduced at the 3′-position by treatment of the compound of formula (VI) with an appropriate agent e.g. an alkali metal azide such as sodium azide e.g. in an organic solvent such as dimethylformamide.

In stage g) the removal of the 5′-protecting group of formula (VII) is advantageously effected by treatment with an appropriate base e.g. sodium bicarbonate.

Alternative B

With regard to stage (a), the 1- and 2-hydroxy groups of the xylose may be blocked with an isopropylidene group, but any blocking group or groups may be used which can be selectively removed later without

deblocking the 3-and 3-hydroxy groups. In the case of the isopropylidene group, blocking of the 1- and 2-hydroxy groups may be effected, for example, by the method of B.R. Baker et al as described in J. Amer. Chem. Soc. 77, 5900 (1955). In lieu of step (a), 1,2-0-isopropylidene- α-D-xylofuranose may be obtained commercially from the Aldrich Chemical Co. or prepared from D-glucose by the following route:

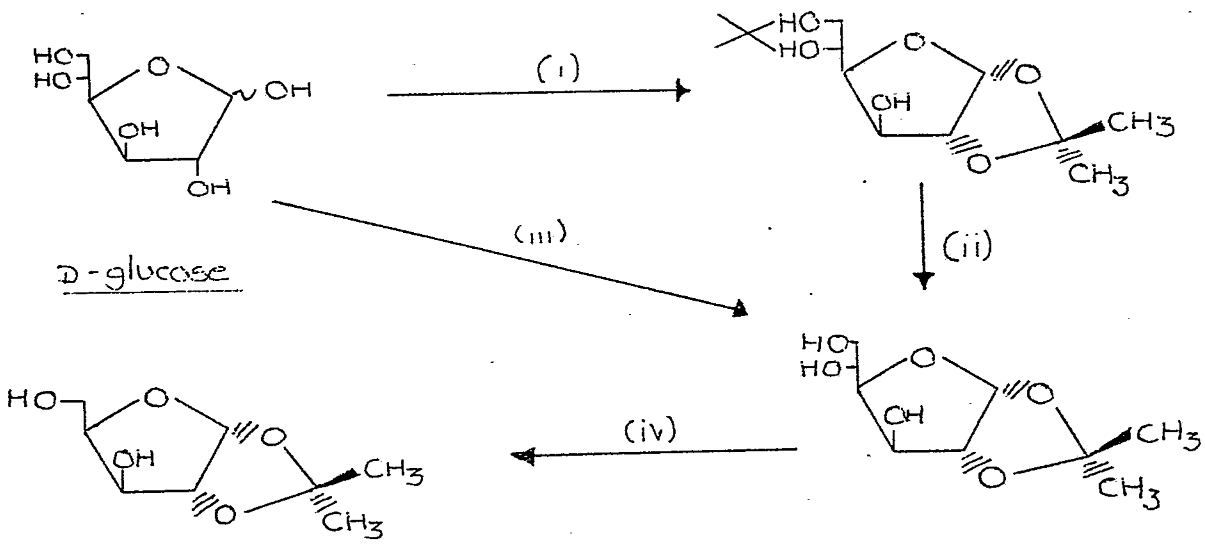


(i) Acetone/HCl (E. Fischer and C. Rund, Ber., 49, 93 (1916)) or acetone/$H_3PO_4$/$ZnCl_2$ (Methods in Carbohydrate Chemistry, R.L. Whistler and M.L. Wolfrom, Eds., II, 320 (Academic Press Inc., New York, 1963)).

(ii) Methanol/$H_2SO_4$ (Methods in Carbohydrate Chemistry, R. L. Whistler and M.L. Wolfrom, Eds., II, 322 (Academic Press Inc., New York, 1963)).

(iii) Acetone/$H_2SO_4$ (Methods in Carbahydrate Chemistry, R.L. Whistler and M.L. Wolfrom, Eds., II, 322 (Academic Press Inc., New York, 1963)).

(iv) (1) $NaIO_4$ (2) $NaBH_4$ (Synthetic Methods in Nucleic Acid Chemistry, W.W. Zorbach and R.S. Tipson, Eds., I, 195 (John Wiley & Sons, New York, 1968)).

With regard to stage (b), the 3- and 5-hydroxy groups are preferably blocked with acyl groups or a methoxycarbonate group, but any blocking groups or group may be used which are/is unaffected by the conditions employed to deblock the 1- and 2-hydroxy groups in option i). In the case of acyl groups, blocking of the 3- and 5-hydroxy groups may be effected, for example, by reacting the compound of formula (II) with an appropriate acyl halide (e.g. chloride) or anhydride, typically in an apolar solvent such as methylene chloride in the presence of bases such as triethylamine and 4-dimethylaminopyridine or in a basic solvent such as pyridine. Suitable acyl blocking groups include acetyl, benzoyl and pivaloyl, the latter being preferred. For pivaloyl blocking groups, the preferred reagent is trimethyacetyl chloride. An alternative blocking group is benzyl, in which case the preferred reageent is a benzyl halide, e.g. benzyl chloride.

With regard to stage (b) option i), the selective deblocking of the 1- and 2-hydroxy groups may be effected, for example, by heating the 3- and 5-protected compound in an aprotic solvent in the presence of an acid catalyst. For the case where A/B is isopropylidene and the 3- and 5-protecting groups are pivaloyl groups, selective deblocking may be effected, for example, by refluxing the compound in a mixture of dioxan and 2% aqueous sulphuric acid. Formation of the cyclic sulphite may be effected, for example, by reacting the 1-, 2-deprotected compound with thionyl chloride or thionyl bromide in an aprotic solvent such as ether in the presence of a base such as pyridine.

With regard to stage (c), the activating groups of the thymine derivative of formula (III) are preferably trimethylsilyl groups, but any suitable blocking groups may be used which can be selectively removed during the course of base/sugar linking without deblocking the 3′- and 5′-hydroxy groups of the xylofuranosyl moiety. In the case of trimethylsilyl groups, blocking of the hydroxy groups of thymine may be effected, for example, by refluxing the thymine or hexamethylenedisilazane in the presence of ammonium sulphate or by refluxing in bis(trimethylsilyl)acetamide (BSA) or by heating the thymine with trimethylsilyl chloride in an aprotic solvent such as benzene in the presence of a base such as triethylamine.

The reaction of the cyclic sulphite protected sugar moiety and the protected thymine derivative of formula (III) to form the compound of formula (IV) may be effected, for example, by heating the two compounds under nitrogen at an elevated temperature for several hours, typically at a temperature of from 80° to 150°C for a period of from 1 to 144 hours, and treating the product, preferably in situ, with an agent which selectively deblocks the hydroxy groups of the thymine moiety without deblocking the 3′- and 5′-hydroxy groups of the

xylofuranosyl moiety to give the compound of formula (IV). For the case where X and Y are trimethylsilyl groups, selective deblocking may be effected, for example, by treatment with a polar solvent such as $NaHCO_3$ in methanol.

Deblocking of the 3′- and 5′-hydroxy groups of the xylofuranosyl moiety may be effected, for example, by treating the compound of formula (IV) with ammoniacal alcohol, typically at a temperature of from 0° C to room temperature, or with a Group I or II metal alkoxide, carbonate, or bicarbonate (or two or more thereof) in a polar solvent such as methanol, typically at a temperature of from 0° C to reflux, and neutralising the product, for example, with an acidic resin.

With regard to stage d) option i), the 3′- and 5′- hydroxy groups are preferably blocked by a ketal group, for example, an acetonide group, or other such groups including, for example, cyclic carbonate, and silyl ether groups, e.g. tetraisopropyldisiloxan-1,3-diyl. In the case of an acetonide group, blocking may be effected, for example, by reaction with acetone, advantageously in the presence of a strong organic or inorganic acid such as hydrochloric acid and preferably in the hence of 2,2- dimethoxypropane. In the case of the tetraisopropyldisiloxan-1,3-diyl group, blocking may be effected, for example, by reaction with 1,3-dichloro-1,1,3,3-tetraisopropyldisiloxane using the method described by W.T. Markiewicz and M. Wiewiorowski in "Nucleic Acid Chemistry", Part 3, L.B. Townsend and R.S. Tipson, Eds., John Wiley and Sons, New York (1986).

1) The leaving group is advantageously an organosulphonyloxy group, for example, an alkyl- or aryl-sulphonyloxy group such as methanesulphonyloxy, p-toluenesulphonyloxy, or p-trifluoromethyl sulphonyloxy, the compound being typically prepared by treatment of a precursor with an appropriate acid halide (e.g. chloride) or anhydride, advantageously at room temperature in the presence of an aprotic base such as pyridine.

2) The cyclisation of the compound is generally effected in the presence of $K_2CO_3$ or a strong base such as 1,8-diazabicyclo[5,4,0]undec-7-ene (DBU) or 1,5-diazabicyclo[4.3.0] non-5-ene (DBN), conveniently in a polar aprotic solvent such as dimethylformamide or acetonitrile. The reaction may also be carried out in the presence of $NaHCO_3$ in a protic solvent, such as methanol or ethanol.

2 option b)

The compound is treated with an agent serving to introduce a reduceable group at the 2′- position. The group is advantageously a halo atom, particularly bromo or iodo, but may alternatively be a thio (-SH), alkylthio (-SR in which R is a $C_{1-6}$ alkyl group), or arylthio group (e.g. phenyl optionally substituted by $C_{1-6}$ alkyl or $C_{1-6}$ alkoxy). The halo group may be introduced by treatment of the compound with an inorganic halide, for example, an alkali metal bromide such as sodium or lithium bromide, or an acyl bromide, for example, acetyl or benzoyl bromide, in which latter case the 3′- and 5′- blocking groups are thereby converted to the corresponding acyl blocking groups, or by reaction with a hydrohalide salt of a tertiary amine, for example, pyridine hydrobromide. The reaction is generally effected in an appropriate organic solvent, for example, acetonitrile or dimethyl formamide, but acetic acid is the preferred solvent when using acetyl bromide.

The resulting compound is then preferably deblocked at the 3′- and 5′-positions, for example, by hydrolysis. In the case of a ketal blocking group, the hydrolysis is advantageously effected under acidic conditions, for example, in the presence of a strong acid such as hydrochloric acid. In the case of ester blocking groups such as acyl or cyclic carbonate blocking groups, the hydrolysis is advantageously effected under basic or acidic conditions, for example using hydrogen chloride in a solvent such as methanol, while in the case of silyl ether groups, such hydrolysis may be effected, for example, using tetrabutylammonium fluoride.

The reduction of the resulting compound particularly when the reduceable group is a halo atom and preferably when deblocked as described above, is conveniently effected by catalytic hydrogenation, for example in the presence of a palladium catalyst. When the reduceable group is a thio or alkylthio group, reduction is advantageously effected by hydrogenation in the presence of Raney nickel.

Alternatively, if 3′ and 5′-deblocking is not effected before the reduction step it can be carried out after reduction, for example, by appropriate hydrolysis as described above.

In a modification of the process outlined above, a compound wherein the 2′-leaving group is a group of formula -OCSOR or -OCSSR (in which R represents a $C_{1-4}$ alkyl group, e.g. methyl, or aryl group, e.g. phenyl) or $-COCOOCH_3$, may be converted directly to a 2′-deoxy compound by the Barton Reaction (D.H.R. Barton et al, J. Chem. Soc., Perkin Transactions I, 1574 (1975), for example by treatment with a trialkyl (e.g. methyl) tin hydride in the presence of an initiator such as azoisobutyronitrile (AIBN).

With regard to step (d), i) a compound of formula (IV) may be deblocked by for example hydrolysis and treated to block the 3′- and 5′-hydroxy groups of 1-(β-D-xylofuranosyl)thymine by a tetraisopropyldisiloxan-1,3-diyl group, for example, by reaction with 1,3-dichloro-1,1,3,3- tetraisopropyldisiloxane using the method of Markiewicz and Wiewiorowski referred to above. Blocking by an aliphatic ketal group (as defined) may be effected by reaction with the appropriate ketone, for example, acetone in the case of the isopropylidine group, advantageously in the presence of a strong organic or inorganic acid such as hydrochloric acid and preferably in the presence of 2,2-dimethoxypropane.

Alternatively with regard to step (d), i), 2a) the preparation of compounds of formula (V) is effected as hereinbefore described, the opening of the anhydro ring being effected by base hydrolysis, preferably in the presence of dilute aqueous sodium hydroxide.

With regard to step (d), i), 2b) the reduceable group is introduced to the 2′-position of a compound of formula (IV) or (V) and is advantageously a benzoate or substituted benzoate group, for example, a

4-chlorobenzoate or 3-trifluoromethylbenzoate group, the compounds being typically prepared by treatment of the corresponding precursor with an appropriate acid halide (e.g. chloride) or anhydride, advantageously at room temperature in the presence of an aprotic base such as pyridine.

With regard to step d), ii), the photolytic removal of the 2′-reduceable group is typically carried out by irradiating the compound with a high pressure mercury lamp having a Pyrex filter in the presence of an electron transfer agent such as N-methylcarbazole or N-ethylcarbazole in an aqueous/organic solvent system such as water/THF (1:10) or water/isopropanol (1:10) at about room temperature under an inert atmosphere such as nitrogen. A catalytic amount of magnesium per chlorate may optionally be present. (J. Saito et al; J. Amer. Chem. Soc. 108, 3115 (1986)).

The compound may then be deblocked at the 3′- and 5′- positions by, for example, hydrolysis. In the case of the tetraisopropyldisiloxan-1,3-diyl blocking group, hydrolysis may be effected using tetrabutylammonium fluoride, preferably in tetrahydrofuran. In the case of an aliphatic ketal blocking group (as defined), for example, the isopropylidene group, hydrolysis is advantageously effected under acidic conditions, for example, in the presence of a strong acid such as hydrochloric acid.

Alternatively, deblocking of the 3′- and 5′- hydroxy groups may occur before deoxygenation and the subsequent irradiation may be carried out using the methods described above.

With regard to step (e), the 5′- blocking agent may be an appropriate halide, for example, trityl chloride. The agent serving to introduce the leaving group in the compound may also be an appropriate halide, such as mesyl chloride. The second agent is preferably added to the 5′-blocked intermediate in situ. Where either agent is a halide, the addition is preferably made in the presence of a base such as pyridine. The introduction of the 3′-azido group (stage f) may be effected, for example, using an alkali metal azide, for example lithium or sodium azide. Finally, with regard to step (g), the deblocking of the 5′-hydroxy group may be effected, for example, by acid hydrolysis, e.g. in aqueous hydrochloric acid.

It should be appreciated that the invention is concerned not only with the overall synthetic route outlined above for the conversion of D-xylose into zidovudine, but also with the individual reaction stages. The overall synthetic route and also its novel individual stages, alone or in combination, each represent further features of the invention together with the novel intermediates referred to below.

a) The novel compounds of formula (IV) wherein :

i) $R^1$ is methoxycarbonyl, $R^2$ is mesylate and $R^4$ is acetyl, hydrogen or trifluoromethylbenzoyl;

ii) $R^1$ and $R^2$ are pivaloyl and $R^4$ is hydrogen;

iii) $R^1$ and $R^2$ together form a tetraisopropyldisiloxan-1,3-diyl group and $R^4$ is trifluoromethylbenzoyl.

b) The novel compounds of stage b) wherein :

i) the 5-hydroxy blocking group is methoxycarbonyl, the 3-leaving group is mesyl the 2-reduceable group is trifluoromethylbenzoyl and the 1-hydroxy blocking group is methyl or acetyl;

ii) the 3- and 5- hydroxy blocking groups are pivaloyl and the 1- and 2- positions are protected by a cyclic sulphite group or are both hydroxy groups.

c) The novel compounds of formula (VI) wherein :

i) $R^1$ is methoxycarbonyl and $R^2$ is mesyl;

ii) $R^1$ and $R^2$ together form tetraisopropyldisiloxan-1,3-diyl.

d) The novel compounds of stage d) wherein :

i) the 5′-hydroxy blocking group is methoxycarbonyl and 2′- and 3′ leaving groups are mesyl;

ii) the 5′-hydroxy blocking group is methoxycarbonyl, the 3′- leaving groups is mesyl and the 2′-position forms a cyclic link with the 2- position of the base;

iii) the 5′-hydroxy blocking group is methoxycarbonyl, the 3′- leaving group is mesyl and the 2′-reduceable group is bromo;

iv) the 3′- and 5′- hydroxy groups are blocked by an acetonide group and the 2′- position forms a cyclic link with the 2-position of the base;

v) the 3′- and 5′- hydroxy groups are blocked by acetyl groups and the 2′- reduceable group is bromo.

(e) The novel compound of formula (VII) wherein $R^1$ is methoxycarbonyl.

f) The use of each and every compound listed in a) - e) above in the synthesis of zidovudine particularly from the starting material D-xylose.

g) A compound as listed in a) - e) above for use in the synthesis of zidovudine particularly from the starting material D-xylose.

h) A process for the preparation of zidovudine from a compound of formula (VII) wherein $R^1$ is methoxycarbonyl.

i) A process for the preparation of zidovudine involving photochemical 2′-deoxygenation of a compound of formula (IV) wherein $R^4$ is a photolytic group.

j) A process for the preparation of zidovudine from D-xylose involving stages a) - g) above, and each and every novel stage thereof.

The following Examples illustrate the present invention.

Examples for Alternative A

Photochemical Method

Example A1 :

Methyl-3-0-mesyl-5-0-(methoxycarbonyl)-2-0-(m-trifluoromethylbenzoyl)-D-xylofuranoside

A flame dried 250ml flask equipped with an addition funnel, magnetic stirrer and nitrogen inlet was charged with methyl 3-0-mesyl-5-0-(methoxycarbonyl)-D-xylofuranoside (5.84g, 19.4 mmoles) (C.D. Anderson, L.Goodman and B.R. Baker, J.Am Chem. Soc. 1958, 80, 5247) and anhydrous pyridine. The solution was cooled in an ice bath and 3-(trifluoromethyl)-benzoyl chloride (5.27g, 25.3 mmoles) was added dropwise over 10 min. After the addition was complete the ice bath was removed and the reaction was continued 24 hours at ambient temperature. The mixture was poured into ice water (400mL) and the aqueous solution extracted with ether (2x250mL). The combined ether extracts were washed with 400mL. of 1.2NHCl and 400mL of 10% aqueous $NaHCO_3$. The solvent was removed by rotary evaporation and the residual oil was purified by flash chromatography (3-inch diameter column containing 18 inches of flash silica gel). The column was eluted with 1:1/ethyl acetate:hexanes while collecting 200mL fractions. Fractions 12-15 contained pure product and these fractions were concentrated in vacuo to afford 8.92g (97.3%) of methyl 3-0-mesyl-5-0-(methoxycarbonyl)-2-0-(m-trifluoromethylbenzoyl)-D-xylofuranoside as a colourless oil containing an approximately equal mixture of α and β anomers. $^1H$ NMR($CDCl_3$) δ 3.09 and 3.19(s,3H,$OCH_3$), 3.34 and 3.37(s,3H,$SO_2CH_3$), 3.79(s,3H,$OCO_2CH_3$), 4.4-4.9(m, 3H,C-4H and C-5H), 5.1-5.6(m,3H,C-1H,C-2H and C-3H) and 7.5-8.5 (m,4H,ArH); TLC $R_f$=0.77 and 0.84, silica gel, $CHCl_3$:$CH_3OH$/90:10.

Example A2 :

1-0-Acetyl-3-0-mesyl-5-0-(methoxycarbonyl)-2-0-(m-trifluoromethylbenzoyl) D-xylofuranose

A 250mL flask equipped with a nitrogen inlet, magnetic stirrer and addition funnel was charged with methyl-3-0-mesyl-5-0-(methoxycarbonyl)-2-0-(m-trifluoromethylbenzoyl)-D-xylofuranoside (19.0g, 40.2mmoles) from Example A1, acetic acid (170mL) and acetic anhydride(25mL). The solution was cooled to 10°C in an ice bath and sulfuric acid (15mL) was added dropwise over 20 minutes. After the additon was complete the ice bath was removed and the reaction was continued for 16 hours at ambient temperature. The reaction solution was poured into a solution of ice water (1.1L) and methanol (0.1L). Stirring was continued an additional 30 minutes and the aqueous solution was extracted with chloroform (3x 250mL). The combined chloroform extracts were carefully washed with 10% aq. $NaHCO_3$ (3x700mL) and the solvent was removed in vacuo to a constant weight affording 19.6g (97.4%) of 1-0-acetyl-3-0-mesyl-5-0- (methoxycarbonyl)-2-0-(m-trifluoromethylbenzoyl)-D-xylofuranose as an approximately equal mixture of anomers :
$^1H$ NMR($CDCl_3$) δ 2.13 and 2.19(s,3H,$OCOCH_3$), 3.13 and 3.21(s,3H,$SO_2CH_3$), 3.78 and 3.79(s,3H,$OCO_2CH_3$), 4.2-4.9(m,3H,C-4H and C-5H), 5.2-5.6(m,2H,C-2H and C-3H), 6.35(s,1H,C-1,β-anomer), 6.57(d,J=4.1Hz,C-1H,α-anomer), 7.5-8.2(m,4H,ArH).

Example A3 :

1-(3-0-Mesyl-5-0-(methoxycarbonyl)-2-0-(m-trifluoromethylbenzoyl)-β-D-xylofuranosyl)thymine

A flame dried 250mL flask equipped with a magnetic stirrer, nitrogen inlet and additional funnel was charged with 1-0-acetyl-3-mesyl-5-0-(methoxycarbonyl)-2-0-(m-trifluoromethylbenzoyl)-D-xylofuranose (19.4g, 38.8mmoles) from Example A2, 2,4-bis (trimethylsilyl)thymine (14.7g, 54.3mmoles) and methylene chloride (200ml). Stannic chloride (62.4mmoles, 1M in methylene chloride) was added dropwise over 25 minutes and the solution was stirred for 20 hours at room temperature. The solution was carefully poured into 15% aqueous $NaHCO_3$ (1.2L)and the aqueous layer extracted with methylene chloride (3x300mL). The combined organic extracts were washed with 10% aqueous $NaHCO_3$ (200mL) and concentrated by rotary evaporation. The residual solid was purified by flash chromatography (70:30/ethyl acetate:hexanes) affording 12.8g (52.0%) of 1-(3-0-mesyl-5-0-(methoxycarbonyl)-2-0-(m- trifluoromethylbenzoyl)-β-D- xylofuranosyl)thymine :
mp: 94-96°C; TLC $R_f$=0.61, silica gel, $CHCl_3$:$CH_3OH$/90:10;

Example A4 :

1-[2-Deoxy-3-0-mesyl-5-0-methoxycarbonyl-β-D-threo-pentofuranosyl]thymine

A 500mL pyrex photochemical reaction vessel equipped with a nitrogen inlet, magnetic stirrer, reflux condenser, quartz immersion well and a 450 watt medium pressure mercury-vapor immersion lamp surrounded by a uranium absorption sleeve was charged with 90% aqueous isopropyl alcohol(500mL), N-methyl carbazole (1.46g, 8.05mmoles) and 1-(3-0-mesyl-5-0-(methoxycarbonyl)-2-0-(m-trifluoromethylbenzoyl)-β-D-xylofuranosyl)thymine (3.80g, 6.71mmoles) from Example A3. After bubbling nitrogen through the solution for 45 minutes the solution was photolyzed for 20 hours. The solvent was concentrated to a 50mL volume by rotary evaporation and the remaining liquid was diluted with chloroform (300mL). The organic solution was washed with 10% aqueous $NaHCO_3$ (2x200ml) and the solvent was removed by rotary

evaporation . The residual oil was purified by flash chromatography (3-inch diameter column containing 15 inches of flash silica gel). The column was eluted with 95:5/$CHCl_3$:$CH_3OH$ while collecting 125mL fractions. Fractions 15-19 contained pure product and these fractions were concentrated in vacuo affording 1.75g (68.9%) of 1-[2-deoxy-3-0-mesyl-5-0-methoxycarbonyl-β-D-threo-pentofuranosyl] thymine as a white crystalline solid.
mp: 70-72°C;
TLC $R_f$=0.51 . Silica gel, $CHCl_3$ : $CH_3OH$/90/10

Chemical Method

Example A5 :

2′-0-Acetyl-3′-0-mesyl-5′-0-(methoxycarbonyl)-1-β-D-xylofuranosylthymine

A flame-dried 250-ml round bottom flask equipped with a magnetic stirrer, addition funnel and nitrogen inlet was charged with 1,2-di-0-acetyl-3-0-mesyl-5-0-methoxycarbonyl-D-xylofuranose (5.0g, 13.4mmoles), prepared as described by C.D. Anderson et al (J.Am. Chem. Soc. 1958, 80 5247; 5.00g) and 2,4-bistrimethylsilyl)thymine (5.43g, 20.1mmoles) in 100 mL of methylene chloride. Stannic chloride (1M in methylene chloride; 20mL) was added dropwise over 30 minutes and the solution was stirred an additional 18h at room temperature. The solution was diluted with 100mL of methylene chloride, transferred to a separatory funnel and carefully washed with 2 x 150mL portions of 10% aq. sodium bicarbonate. The organic solution was filtered through celite and concentrated by rotary evaporation. The crude product was purified by flash chromatography (97:3/$CHCl_3$:$CH_3OH$) affording 3.85g (65.8%) of 2′-0-acetyl-3′-0-mesyl-5′-0-(methoxycarbonyl)-1-β-D-xylofuranosylthymine: mp 87-89°C; $^1H$ NMR ($CDCl_3$)δ 1.94 (d,j=1.18Hz,3H,-C-5$CH_3$), 2.15 (s,3H,$COCH_3$), 3.17 (s,3H,$SO_2CH_3$), 3.83 (s,3H,$CO_2CH_3$), 4.50 (m, 3H, C-4′ and C-5′H), 5.14 (m, 1H, C-3′H), 5.25 (m, 1H, C-2′H), 6.09 (d, J=3.37HZ, 1H, C-1′H), 7.32 (m, 1H, C-6H) and 8.64 (broad, 1H, NH); $^{13}C$ NMR ($CDCl_3$) δ 12.58, 20.53 38.49, 55.33, 64.17, 76.46 79.36, 79.82, 87.27 112.01, 134.73, 150.48, 155.13, 163.74 and 169.69; TLC, $R_f$ = 0.59, silica gel, $CHCl_3$:$CH_3OH$/90:10.

Example A6 :

3′-0-Mesyl-5′-0-(methoxycarbonyl)-1-β-D-xylofuranosylthymine

Anhydrous methanolic HCl was prepared by adding acetyl chloride (4.42g) dropwise to anhydrous methanol (0.125 l) at room temperature. 2′-0-acetyl-3′-0-mesyl-5′-0-(methoxycarbonyl)-1-β-D-xylofuranosylthymine (3.50g, 8.02mmoles) from Example A5 was added and the resulting solution was stirred 3 days at ambient temperature. The solution was cooled 1 hour in an ice bath, the resulting precipitated solids collected by filtration and washed with 10mL of prechilled methanol. The solids were dried 18h in a vacuum at 50°C affording 2.45g (75.2%) of 3′-0-mesyl-5′-0-(methoxycarbonyl)-1-β-D-xylofuranosylthymine:
mp = 163-166°C, $^1H$ NMR ($CDCl_3$) δ 1.91 (d, J = 0.94Hz, 3H, C-5 $CH_3$), 3.10 (s, 3H, $SO_2CH_3$), 3.49 (broad, 1H, OH), 3.83 (s, 3H, $CO_2CH_3$), 4.49-4.75 (m, 4H, C-2H, C-4′-H, C-5′-H), 5.13 (doublet of doublets, J = 4.38Hz and 3.04Hz, 1H, C-3′H), 5.84 (d, J = 2.64Hz, 1H, C-1′H), 7.50 (m, 1H, C-6H), and 10.01 (broad, 1H, NH); $^{13}C$ NMR (DMSO-$d_6$) δ 12.07, 37.58, 54.91, 65.10, 76.39, 76.75, 82.16, 88.43, 109.56, 135.34, 150.48, 154.75 and 163.56; TLC, $R_f$ = 0.53, silica gel, $CHCl_3$: $CH_3OH$/90:10; Elemental Analysis calcd C, 39.59; H, 4.60; N, 7.10. Found C, 39.41; H, 4.67; N, 7.01.

Example A7

3′-0-Mesyl-5′-0-(methoxycarbonyl)-1-β-D-xylofuranosylthymine

Sodium acetate (0.563g, 6.87mmoles) and hydroxylamine hydrochloride (0.477g, 6.87mmoles) were dissolved in pyridine (10mL) in a 25ml flask equipped with a magnetic stirrer and nitrogen inlet. The solution was stirred 30 minutes as room temperature followed by the addition of 2′-0-acetyl-3′-0-mesyl-5′-0-(methoxycarbonyl)-1-β-D- xylofuranosylthymine (1.0g, 2.29mmoles) from Example A5. After stirring 20 hours at room temperature the solution was poured into a solution of 50mL of ethanol and 10mL of acetone. The solution was filtered and concentrated by rotary evaporation. Residual pyridine was removed by concentration from an additional 40mL of ethanol. The residual oil was triturated with 20mL of ethanol at 0°C for 30 minutes and the precipitated solids were collected by filtration and dried 18 hours in a vacuum oven at 50°C affording 0.662g (73.0%) of 3′-0-mesyl-5′-0- (methoxycarbonyl)-1-β-D-xylofuranosylthymine:
mp = 162-165°C;
TLC $R_f$ = 0.53, Silica Gel, ($CHCl_3$ : $CH_3OH$ 90:10)

The produce was chromatographically and spectroscopically identical to material synthesized in Example A6.

Example A8

3′-0-mesyl-5′-0-(methoxycarbonyl)-1-β-D-xylofuranosylthymine

2′-0-Acetyl-3′-0-mesyl-5′-0-(methoxycarbonyl)-1-β-D-xylofuranosylthymine (1.0g, 2.29mmoles) from Example A5 was dissolved in a mixture of pyridine (8mL) and acetic acid (2mL). After stirring 48 hours at room temperature, the solution was poured into a solution of ethanol (50ml) and of acetone (10mL). The solution was concentrated by rotary evaporation and residual pyridine was removed by concentration from an additional 40mL of ethanol. The residual oil was triturated with 20mL of ethanol at 0°C for 1 hour and the precipitated solids were collected by filtration and dried 18 hours in a vacuum oven at 50°C affording 554mg (61.3%) of 3′-0-mesyl-5′-0-(methoxycarbonyl)-1-β-D- xylofuranosylthymine:
mp = 162-165°C;
TLC $R_f$ = 0.53 Silica Gel, ($CHCl_3$ : $CH_3OH$ 90:10)

The product was chromatographically and spectroscopically identical to material synthesized in Example A6.

Example A9

2′-Bromo-2′-deoxy-3′-0-mesyl-5′-0-(methoxycarbonyl)-1-β-D-xylofuranosyl thymine

A 25 mL flask equipped with an addition funnel, magnetic stirrer and nitrogen inlet was charged with 3′-0-mesyl-5′-0-(methoxycarbonyl)-1-β-D-xylofuranosylthymine (0.05g,1.27mmoles) from Example A7, triphenylphosphine (0.663g, 2.53mmoles) and DMF (10mL). A solution of diisopropyl azodicarboxylate (0.512g, 2.53mmoles) in DMF (1mL) was added dropwise over 10 minutes and stirring was continued an additional 45 minutes. Lithium bromide (0.22g, 2.53mmoles) and sodium bisulfate monohydrate (0.349g, 2.53mmoles) were added and the suspension was heated at 110°C for an additional 45 minutes. The suspension was cooled to room temperature, diluted with ethyl acetate and washed with 2x100mL portions of water. Solvent was removed by rotary evaporation and the residual solids were purified by flash chromatography (96:4 $CHCl_3$:$CH_3OH$). The appropriate fractions were combined and concentrated in vacuo affording 0.459g (79.0%) of 2′bromo-2′-deoxy-3′-0- mesyl-5′-0-(methoxycarbonyl)-1-β-D-xylofuranosylthymine :
mp = 86-89°C;
TLC, $R_f$ = 0.77, Silica Gel, 90:10 $CHCl_3$ : $CH_3OH$

The product was chromatographically and spectroscopically identical to material synthesized in Example A12 below.

Example A10

2′,3′-Di-0-mesyl-5′-0-(methoxycarbonyl)-1-β-D-xylofuranosylthymine

A flame dried, three necked 250mL flask equipped with a magnetic stirrer, nitrogen inlet and addition funnel was charged with 3′-0-mesyl-5′-0-(methoxycarbonyl)-1-β-D-xylofuranosylthymine (5.00g, 12.7mmoles) from Example A6, triethylamine (3.23g, 31.9mmoles) and methylene chloride (100mL). The solution was cooled in an ice bath, a solution of methanesulfonyl chloride (3.05g, 26.6mmoles) in 5mL of methylene chloride added dropwise over 20 minutes and stirring continued an additional 1h. The solution was transferred to a separatory funnel, washed with 100mL portions of water, 1.2N hydrochloric acid and 10% aqueous sodium bicarbonate and concentrated by rotary evaporation. The resulting crude solid was purified by flash chromatography (94:6; $CHCl_3$:$CH_3OH$) affording 4.31g (72.0%) of 2′,3′-di-0-mesyl-5′-0-(methoxycarbonyl)-1-β-D-xylofuranosylthymine: mp 89-91°C; $^1H$ NMR ($CDCl_3$) δ 1.94 (d, J = 1.08Hz, 3H, C-5 $CH_3$), 3.14 (s, 3H, $SO_2CH_3$), 3.25 (s, 3H, $SO_2CO_3$), 3.84 (s, 3H, $CO_2CH_3$), 4.55-4.70 (m, 3H, C-4′H and C-5′H), 5.25-5.31 (m, 2H, C-2′H AND C-3′H), 5.99 (d, J = 3.27Hz, 1H, C-1′H), 7.38 (m, 1H, C-6H) and 8.67 (broad, 1H, NH); $^{13}C$ NMR ($CDCl_3$) δ 12.49, 38.22, 38.52, 55.37, 63.94, 78.51, 79.47, 83.70, 88.23, 111.64, 134.70, 151,09, 155.13 and 163.90; TLC, $R_f$ = 0.58, silica gel, $CHCl_3$:$CH_3OH$/90:10.

Example A11

2,2′-Anhydro-3′-0-mesyl-5′-0-(methoxycarbonyl)-1-β-D-lyxofuranosylthymine

A mixture of 2′, 3′-di-0-mesyl-5′-0-(methoxycarbonyl)-1-β-D-xylofuranosyl thymine (4.00g, 8.47mmoles) from Example A10, and potassium carbonate (3.52g, 25.4mmoles) in acetonitrile (80mL) was heated 4h at reflux. The solution was filtered, salts washed with 2 x 50mL portions of $CHCl_3$ and solvent removed by rotary evaporation affording 3.18g (100%) of 2,2′-anhydro-3′-0-mesyl-5′-0- (methoxycarbonyl)-1-β-D- lyxofuranosylthymine as a white solid: mp 209-211°C; $^1H$ NMR (DMSO-$d_6$) δ 1.81 (d, J = 1.07Hz, 3H, C-5 $CH_3$), 3.35 (s, 3H, $SO_2CH_3$), 3.66 (s, 3H, $CO_2CH_3$), 3.96-4.40 (m, 2H, C-5′H), 4,63 (m, 1H, C-4′H), 5.53-5.70 (m, 2H, C-2′H and C-3′H), 6.19 (d, J = 3.40Hz, 1H, C-1′H) and 7.77 (m, 1H, C-6H); $^{13}C$ NMR (DMSO-$d_6$) δ 13.39, 37.64, 54.86, 64.80, 75.97, 77.03, 79.48, 88.75, 117.03, 131.83, 154.57, 159.42 and 171.21; TLC, $R_f$ = 0.21, silica gel, $CHCl_3$: $CH_3OH$/90:10.

Example A12

2′-Bromo-2′deoxy-3′-0-mesyl-5′-0-(methoxycarbonyl)-1-β-D-xylofuranosylthymine

A mixture of 2,2′-anhydro-3′-0-mesyl-5′-0-(methoxycarbonyl)-1-β-D-lyxofuranosyl-thymine (0.50g, 1.33mmoles) from Example A11, lithium bromide (0.231g, 2.66 mmoles) and sodium bisulfate monohydrate (0.184g, 1.33mmoles) in acetonitrile (15mL) was heated 4.5h at reflux. The solution was cooled to room temperature, diluted with chloroform, washed with water and solvent removed by rotary evaporation affording 0.561g (93.4%) of 2′-bromo-2′-deoxy-3′-0-mesyl-5′-0-(methoxycarbonyl)-1-β-D-xylofuranosylthymine: mp 84-88°C; $^1$H NMR ($CDCl_3$) δ 1.95 (d, J = 1.23Hz, 1H, C-5 $CH_3$), 3.11 (s, 3H, $SO_3CH_3$), 3.85 (s,3H, $CO_2CH_3$) 4.47-4.81 (m, 4H, C-2′H, C-4′H and C-5′H), 5.27 (doublet of doublets, J = 4.17Hz and 3.24Hz, 1H, c-3′H) 6.32 (d, J = 3.84Hz, 1H, C-1′H), 7.38 (q, J = 1.23Hz, 1H, C-6H) and 8.24 (broad, 1H, NH); $^{13}$C NMR (DMSO-$d_6$) δ 12.06, 38.61, 48.77, 54.95, 64.91, 76.59, 82.01, 88.64, 109.98, 134.56, 150.37, 154.71 and 163.48; tlc, $R_f$ = 0.77, silica gel, $CHCl_3$: $CH_3OH$/90:10.

Example A13

1-[2-Deoxy-3-0-mesyl-5-0-methoxycarbonyl-β-D-threo-pentofuranosyl]-thymine

A flame dried 3-necked 100-mL round bottomed flask equipped with a nitrogen inlet, addition funnel and reflux condenser was charged with 2′-bromo-2′ deoxy-3′-0-mesyl-5′-0- (methoxycarbonyl)-1-β-D-xylofuranosylthymine (0.500g, 1.09mmoles) from Example A12 and toluene (50mL). The solution was warmed to 85°C and neat tributyltin hydride (0.500g, 3.28mmoles) was added dropwise over 1 minute. After approximately one third of the tributyltin hydride was added, a few crystals of azobisisobutyronitrile were added. The solution was heated at 85°C for 90 minutes, cooled to room temperature and solvent removed by rotary evaporation. The residual oil was dissolved in acetonitrile (75mL), the resulting solution washed with 3 x 75 mL portions of hexane and solvent removed by rotary evaporation affording 0.362g (88.9%) of 1-[2-deoxy-3-0-mesyl-5-0-methoxycarbonyl-β-D-threopentofuranosyl]-thymine as a white crystalline solid: mp 70-74°C; $^1$H NMR ($CDCl_3$) δ 1.95 (d, J = 1.21, 3H, C-5 $CH_3$), 2.51-2.85 (m, 2H, C-2′H), 3.07 (s, 3H, $SO_2CH_3$), 3.82 (s, 3H, $CO_2CH_3$), 4.26-4.57 (m, 3H, C-4′ and C-5′H), 5.28 (m, 1H, C-3′H), 6.28 (doublet of doublets, J = 7.63 and 3.88, 1H, C-1′H), 7.43 (m, 1H, C-6H) and 8.79 (broad, 1H, NH); $^{13}$C NMR ($CDCl_3$) δ 12.58, 38.50, 39.33, 55.31, 64.26, 76.40, 79.16, 83.72, 111.48, 135.07, 150.58, 155.24 and 163.75: TLC, $R_f$ = 0.51, silica gel, $CHCl_3$:$CH_3OH$/90:10.

Example A14

3′-Azido-3′-deoxy-5′-0-(methoxycarbonyl)thymidine

A mixture of 1-[2-deoxy-3-0-mesyl-5-0-methoxycarbonyl-β-D-threopentofuranosyl]-thymine (5.0g, 13.2mmoles) from Example A13 and sodium azide (1.07g, 16.5mmoles) in dimethylformamide (40mL) was heated at 85-90°C for 4h. The suspension was cooled to ambient temperature poured into 300mL of water and the aqueous solution extracted with 3 x 100mL portions of ethyl acetate. The combined organic extracts were washed with 2 x 100mL portions of water and concentrated by rotary evaporation affording 3.88g (90.2%) of 3′-azido-3′-deoxy-5′-0-(methoxycarbonyl)thymidine as a colorless oil: $^1$H NMR ($CDCl_3$) δ 1.91 (d, J = 1.15Hz, 3H, C-5-$CH_3$), 2.30-2.49 (m, 2H, C-2′H), 3.83 (s, 3H, $CO_2CH_3$), 3.97-4.44 (m, 4H, C-3′H, C-4′H and C-5′H), 6.21 (t, J = 6.26Hz, 1H, C-1′H), 7.29 (q, J = 1.15Hz, 1H, C-6H) and 8.75 (broad, 1H, NH); $^{13}$C NMR ($CDCl_3$) δ 12.42, 37.57, 55.28, 60.04, 66.39, 81.56, 84.94, 111.38, 135.28, 150.45, 155.18 and 164.03; TLC, $R_f$ = 0.78, silica gel, $CHCl_3$:$CH_3OH$/90:10.

Example A15

3′-Azido-3′-deoxythymidine

A mixture of 3′-azido-3′-deoxy-5′-0-(methoxycarbonyl)thymidine (2.50g, 7.68mmoles) from Example A14 and sodium bicarbonate (0.50g) was heated 2.5h at reflux in methanol (75mL). The solvent was removed by rotary evaporation and the residual semi-solid was triturated with cold 5% aq. sodium bicarbonate (50mL). The resulting solid was collected by filtration and dried in a vacuum oven at 55°C for 17h affording 1.71g (83.4%) of 3′-azido-3′- deoxythymidine:

mp 119-121°C; $^1$H NMR (DMSO-$d_6$) δ 1.79 (d, J = 1.04Hz, 3H C-5 $CH_3$), 2.10-2.56 (m, 2H, C-2′H), 3.31-3.92 (m, 3H, C-3′ and C-5′H), 4.40 (m,1H,C-4′H), 5.19 (5, J = 5.22Hz, 1H, OH), 6.11 (t, J = 6.46Hz, 1H, C-1′H), 7.68 (q, J = 1.04, 1H, C-6H) and 10.65 (broad, 1H, NH); TLC, $R_f$ = 0.35, silica gel, $CHCl_3$: $CH_3OH$/90:10.

Examples for Alternative B

All glassware used in non-air reactions was dried in an oven overnight at 150°C. The glassware was removed from the oven and evacuated and purged with nitrogen three times. The system then remained under a nitrogen atmosphere for the duration of the experiment.

Solvents were purified as follows: Methylene chloride was freshly distilled from phosphorus pentoxide. Benzene was washed with sulphuric acid and distilled prior to use. Triethylamine was distilled one day in advance from potassium hydroxide and stored over potassium hydroxide pellets. Pyridine was distilled one day in advance from potassium hydroxide and stored over potassium hydroxide pellets. Thionyl chloride was freshly distilled from 10% w/w triphenyl phosphite. Trimethysilyl chloride was freshly distilled before use.

Example B1

1,2-0-Isopropylidene-α-D-xylofuranose

D-xylose was converted to 1,2-0-isopropylidene-α-D-xylofuranose by the method of B.R. Baker et al as described in J. Amer. Chem. Soc. 77, 5900 (1955).

Example B2

1,2-0-Isopropylidene-3,5-di-0-pivaloyl-α-D-xylofuranose

A dry 250mL round-bottomed flask was equipped with an internal thermometer and magnetic stirring bar and maintained under a nitrogen atmosphere. The flask was charged with 1,2-0-isopropylidene-α-D-xylofuranose (9.5g, 49.95 mmol) from Example B1, methylene chloride (70mL), and triethylamine (8.7mL, 62.4 mmol). The solution was cooled to an internal temperature of 3°C using a brine/ice bath. Trimethylacetyl chloride (7.69ml, 62.4 mmol) was added dropwise, via syringe, over a ten minute period. Finally, 4-dimethylaminopyridine (1.83g, 14.99 mmol) was added and stirring continued for 75 minutes. The mixture was brought to room temperature and diluted with methylene chloride (70mL). It was then extracted with water (40mL) and brine (40mL). The organic layer was dried over magnesium sulfate, filtered and concentrated in vacuo. The product was purified on a preparatory silica gel column eluted with 80:20 (v/v) hexane/ethyl acetate. The product was obtained as a clear colourless oil. Yield 29%.

TLC (silica gel): Rf 0.63 (80:20 hexane/ethyl acetate)

Calc:

C 60.32, H 8.43

Found:

C 60.25, H 8.45

Example B3

3,5-Di-0-pivaloyl-α-D-xylofuranose

A 250mL round-bottomed flask was charged with 1,2-0-isopropylidene-3, 5-di-0-pivaloyl-α-D-xylofuranose from Example B2, dioxane (75mL), and 2% aqueous sulfuric acid (45mL). The turbid solution was heated at reflux for four hours. The solution was then cooled to room temperature and neutralized using solid sodium bicarbonate. The mixture was concentrated and dried in vacuo. The product was purified using a silica gel flash chromatography column. The product was obtained as a clear yellow tinted oil.

TLC (silica gel): Rf 0.17 (70:30 hexane/ethyl acetate)

Calc:

C 56.59, H 8.23

Found:

C 56.45, H 8.25

Example B4

Cyclic sulphite of 3,5-di-0-pivaloyl-α-D-xylofuranose

The cyclic sulphite was prepared by the method of C.H. Gagnieu et al as described in J. Carb. Chem. 5, 153 (1986). A dry 250mL round-bottomed flask was equipped with an internal thermometer and maintained under a nitrogen atmosphere. The flask was charged with 3,5-di-0-pivaloyl-α-D-xylofuranose (5.3g, 16.6 mmol) from Example B3 and dry diethyl ether (30ml). Thionyl chloride (1.21ml, 16.6 mmol) was added and the solution cooled to 0°C in a brine/ice bath. Pyridine (2.75g, 34.8 mmol) in dry diethyl ether (30mL) was added dropwise over a 10 minute period. Stirring was continued for an additional 35 minutes. The reaction mixture was filtered and the filtrate loaded on to a short silica gel flash chromatography column which was then eluted with 50:50 (v/v) hexane/ethyl acetate.

Example B5

Nucleoside formation

2,4-Bis-0-trimethylsilylthymine was prepared by the method of T.J. Bardos et al as described in J. Org. Chem. 34, 3806 (1969). To a dry one litre round-bottomed flask were added thymine (3.78g, 30 mmol, dried overnight in a vacuum oven at 60°C), benzene (250mL), trimethylsilyl chloride (8.5ml, 60 mmol), and triethylamine (8.35mL). The reaction was heated at reflux, under nitrogen, for three hours. The mixture was cooled to room temperature and filtered through a Schlenk tube. The filtrate was divided into two portions.

One portion of the 2,4-bis-0-trimethysilylthymine and one portion of the cyclic sulphite from Example B4 were combined and the benzene distilled off under nitrogen. The two components were fused at 150°C for seven hours under nitrogen. The reaction was cooled to room temperature and diluted with methylene chloride (25mL) and methanol (10mL). The solution was stirred at room temperature for 30 minutes. The reaction mixture was then concentrated in vacuo and purified on a silica gel flash chromatography column eluted with 98:2 (v/v) chloroform/methanol.

Yield: 38% from 3,5-di-0-pivolyl-α-D-xylofuranose
TLC (silica gel): Rf 0.3 (95:5 v/v chloroform/methanol)

The product was a off-white glassy solid which upon heating turned to a clear solid by 72°C and melted at from 80 to 84°C.

Example B6

1-(β-D-Xylofuranosyl)thymine

Fox J.J., Yung N., Davoroll J. and Brown G.B., J. Amer. Chem. Soc., 1956 78 21117. The nucleoside from Example B5 (157.6mg, 0.37 mmol) was dissolved in methanol (3mL) and sodium bicarbonate (21mg, 0.25 mmol) added. The solution was heated at reflux for 70 minutes. At this point, sodium methoxide (85mg, 1.48 mmol) was added and the solution was heated at reflux for an additional 20 minutes. The solution was then cooled to room temperature and neutralized with Dowex 50W-X8 (acid form). The resin was removed by filtration and washed with methanol. The filtrate was concentrated in vacuo to an orange-tinted foam which crystallised on purification.

Chemical Method

Example B7

1-(3′,5′-0-Isopropylidene-β-D-xylofuranosyl)thymine

Crystalline 1-(β-D-xylofuranosyl)thymine (5.2g 0.020 mole) from Example B6 was stirred vigorously at 20°C in a solution of acetone (20mL) and 2,2-dimethoxypropane (20mL) containing concentrated hydrochloric acid (0.1mL). After two hours, the clear solution was evaporated to afford an off-white foam (6.8g) which crystallised from ethyl acetate (15mL) as white crystals.
Yield: 3.4g (57%), m.p. 179-182°C

The NMR spectrum was in good agreement with the proposed structure.

A considerable quantitiy of isopropylidene derivative remained in the mother liquors.

In a separate experiment, 1-(β-D-xylofuranosyl)thymine (4.4g, 0.017 mole) from Example B6 was converted to crude isopropylidene derivative as an off-white foam (5.0g, 98%) suitable for use in the next step.

Example B8

1-(3′,5′-0-isopropylidene-2′-0-mesyl-β-D-xylofuranosyl)thymine

The crude 3′,5′-0-isopropylidene derivative (5.0g, 0.17 mole) from Example B7 in pyridine (33mL) was allowed to react with methanesulphonyl chloride (3mL, 0.037 mole) at 20°C for 1.5 hours and then at 40°C for 1 hour. Chromatography revealed one major new product.

Evaporation of the solvent gave a yellow residue which was dissolved in chloroform (60mL) and washed with ice cold saturated aqueous sodium hydrogen carbonate (3 x 20mL) and water (1 x 40mL). The chloroform solution was then dried over anhydrous magnesium sulphate. Evaporation of the solvent provided a yellow gum.
Crude yield: 7.8g (123%)

Example B9

1-(2,2′-Anhydro-3′5′-0-isopropylidene-β-D-xylofuranosyl)thymine

The impure 2′-0-mesylate (7.8g, equivalent to 0.017 mole) from Example B8 was heated to 100-110°C in a solution of dimethylformamide and 1,8-diazabicyclo [5,4,0]undec-7-ene (4.0mL, 0.026 mole) for 8 hours. At this time, chromatography revealed a single major new component and no starting material.

High vacuum evaporation afforded a yellow syrup which crystallized from ethyl acetate (30mL) as fine needle crystals. After standing at 4°C for two hours, white crystals were collected.
Yield: 3.9g (82%)

The NMR spectrum of the compound was in good agreement with the proposed structure.

For analytical purposes, the 3.9g of material was further purified by slurrying in hot methanol (15mL) and cooling to 0°C.

After this procedure the weight of fine white needles of the title compound was 2.9g, m.p. 273-274°C.

Example B10

1-(3′,5′-Di-0-acetyl-2′-bromo-2′-deoxy-β-D-xylofuranosyl)thymine

Purified 1-(2,2′-anhydro-3′,5′-0-isopropylidene-β-D-xylofuranosyl) thymine (2.6g, 9.3 mmol) from Example B9 was heated and stirred at 100°C in a solution of acetic acid (25mL) and acetyl bromide (2.5mL, 33.8 mmol). After 45 minutes, the red solution was evaporated to a red glass (4.3g).

Silica gel chromatography (70-230 mesh, 100g) of the product using ethyl acetate:chloroform as eluent (1:1 v/v) afforded a white foam.

Yield: 3.4g (90%)

The compound gave a sharp, well resolved NMR spectrum in agreement with the proposed structure.

Examlpe B11

1-(3'5'-Di-0-acetyl-2'-deoxy-β-D-threo-pentofuranosyl)thymine

Chromatographically purified 1-(3'5'-di-0-acetyl-2'-bromo-2'-deoxy-β-D-xylofuranosyl)thymine (0.5g, 1.23 mmol) from Example B10 in 50% aqueous methanol (8mL) containing sodium acetate trihydrate (0.3g) was hydrogenated over 5% palladium on barium sulphate (0.2g) at NTP.

Over a period of 30 minutes, the vigorously stirred solution absorbed about 50ml of hydrogen. Chromatographic examination of the reaction mixture revealed two major new products and no starting material.

After filtering and evaporation, the residue was dissolved in water (7mL) and extracted with ethyl acetate (3 x 7mL). The combined extracts were dried over anhydrous sodium sulphate and evaporated to a colourless syrup.

Crude yield: 0.32g (79%)

The NMR spectrum of the crude syrup revealed that it consisted of approximately 70% of the required compound (by comparison with the spectrum of an authentic sample) together with an unknown compound(s).

In another experiment, the 2'-bromonucleoside (4.6g, 11.3 mmol) from above was hydrogenated in the usual manner. Part of this material was chromographed on silica gel (110g) using ethyl acetate/ isopropanol (10:1 v/v) as eluent.

The resulting chromatographically homogeneous sample from the column afforded an NMR spectrum in full agreement with the proposed structure.

Example B12

1-(2'-Deoxy-β-D-threo-pentofuranosyl)thymine

Chromatographically purified 1-(3',5-di-0-acetyl-2'-deoxy-β-D-threopentofuranosyl)thymine (0.3g, 0.92 mmol) from Example B12 was warmed to 40° C in methanolic ammonia (4ml, 10% w/v) for three hours.

Evaporation of the solvent afforded a white foam smelling faintly of acetamide and possessing the same chromatographic Rf as an authentic sample of the desired product.

Crude yield: 0.25g (104%)

Photochemical Method

Example B13

1-(3',5'-0-Isopropylidene-β-D-xylofuranosyl)thymine

A 250mL round-bottomed flask was charged with 1-(β-D-xylofuranosyl)thymine (5.2g, 20mmol), acetone (20mL) from Example B6, and 2,2-dimethoxypropane (20mL). To this solution was added conc. HCl (0.1mL). After stirring for two hours at room temperature, the solution was neutralized with potassium carbonate. The brown solution was then filtered and concentrated in vacuo to give a white solid (6.9g). The crude product was absorbed onto 20g of silica gel and loaded onto a 140g silica gel flash column. The column was initially eluted with ethyl acatate/hexanes (4:1 v/v) to remove a minor impurity, followed by ethyl acetate. Fractions containing the product were concentrated in vacuo to give a white solid.

Yield: 5.9g (99%), m.p. 179-181°C

TLC (silica gel): Rf 0.19 (4:1 v/v ethyl acetate/hexanes)

Example B14

1-(3',5'-0-Isopropyldene-2'-0-p-trifluoromethylbenzoyl-β-D-xylofuranosyl)thymine

A dry 250mL 3-necked round-bottomed flask was charged with the nucleoside from Example B13 4.77g, 16mmol), dry methylene chloride (70mL), and dry triethylamine (3.36mL, 24mmol) under nitrogen. The solution was cooled in an ice bath and 3-(trifluoromethyl)benzoyl chloride (5g, 24mmol) was added dropwise over a 10 minute period. DMAP (880mg, 7.2mmol) was then added, whereupon a white precipitate was obtained. After stirring for 35 minutes, methylene chloride (160mL) was added and the resulting solution washed with satd. aqu. sodium bicarbonate (1x50mL) and brine (1x50mL), then dried over anhy magnesium sulphate, filtered and concentrated in vacuo. The crude product was aborbed onto 20g of silica gel and loaded onto a 250g silica gel flash column. The column was eluted with chloroform, followed by chloroform/methanol (90:10 v/v). Fractions containing the product were concentrated in vacuo to give a white solid.

Yield: 79%, m.p. 205-207°C

TLC (silica gel): Rf 0.54 (95:5 v/v chloroform/methanol)

Example B15

1-(3′,5′-0-Isopropylidene-2′-deoxy-β-D-threo-pentofuranosyl)thymine

A solution of the nucleoside from Example B14 (806mg, 1.71mmol) and N-methylcarbazole (336mg, 1.82mmol) in isopropanol/water (10:1 v/v, 286mL) was irradiated with an Hanovia 450W high pressure mercury lamp through a Pyrex filter under nitrogen at room temperature for 4 hours. The solution was then concentrated in vacuo and the residue dissolved in methylene chloride. The resulting solution was washed with satd. aqu. sodium bicarbonate (2x100mL), dried over anhy. magnesium sulphate, filtered and concentrated in vacuo to give a yellow-brown oil. The crude product was absorbed onto 6g of silica gel and loaded onto a 44g flash column. The column was eluted with chloroform/methanol (98:2 v/v). Fractions containing the product were concentrated in vacuo to give a white solid.
Yield: 83.5%
TLC (silica gel): Rf 0.42 (95:5 v/v chloroform/methanol)

Example B16

1-(2′-Deoxy-β-D-threo-pentofuranosyl)thymine

The nucleoside from Example B15 (403.3mg, 1.43 mmol), Dowex 50W-X8 ($H^+$ form, 2.5g), and water (20mL) were heated at 70°C for 1 hour. The mixture was then cooled in an ice bath and the resulting resin filtered off. The filtrate was concentrated in vacuo to give a brown solid (99% yield). This material was absorbed onto 1g of silica gel and loaded onto a 7g flash column. The column was eluted with chloroform/methanol (95:5v/v) and the eluate concentrated in vacuo. Impure fractions were re-chromatographed. The product was obtained as a white crystalline solid.
Yield: 70%, m.p. 166-167°C (lit. m.p. 168-169°C)
TLC (silica gel): Rf 0.27 (90:10 v/v chloroform/methanol)

Example B17

1-(2′-Deoxy-3′-0-mesyl-5′-0-trityl-β-D-threo-pentofuranosyl)thymine

Impure 1-(2′-deoxy-β-D-threo-pentofuranosyl)thymine (0.49g, 2.0 mmol) from Example B16 in pyridine (8ml) was treated with trityl chloride (0.75g, 2.7 mmol) at 40°C for five hours. The solution was cooled to 10-15°C, mesyl chloride (0.23mL, 3.0 mmol) was introduced, and the temperature maintained at 10-15°C for 16 hours. Standard isolation procedures afforded a pale yellow solid which crystallized from ethyl acetate as off-white crystals.
Yield: 0.87g (75%)

Chromatographic and NMR data for the compound were in full agreement with an authentic sample of the title compound.

Example B18

1-(3′-Azido-2′,3′-dideoxy-5′-0-trityl-β-D-erythro-pentofuranosyl)thymine

Crystalline 1-(2′-deoxy-3′-0-mesyl-5′-0-trityl-β-D-threo-pentofuranosyl) thymine (0.80g, 1.4 mmol) from Example B17 was stirred in a solution of DMF (6mL) in the presence of sodium azide (0.10g, 1.54 mmol) at 95°C for 4.5 hours.

The product was isolated by firstly destroying excess azide ions with sodium nitrite (0.10g, 1.5 mmol) dissolved in a solution of 20% aqueous acetic acid (2mL) by stirring for one hour at 20°C. Additional aqueous acetic acid (12mL) was then added to precipitate the product which was isolated by filtration and drying.
Crude yield: 0.60g (84%)

The 3′-azido derivative was characterised by the usual analytical techniques.

Example B19

1-(3′-Azido-2′,3′-dideoxy-β-D-erythro-pentofuranosyl)thymine (zidovudine)

Crude 1-(3′-azido-2′,3′-dideoxy-5′-0-trityl-β-D-erythro-pentofuranosyl) thymine (0.60g, 1.2 mmol) from Example B18 was suspended in a solution of 50% aqueous methanol (5mL) adjusted to pH 2.0 with hydrochloric acid, and refluxed for 4 hours. On cooling to 20°C, triphenylmethanol crystallised out and was removed by filtration. The mother liquors were evaporated to remove most of the alcohol. On cooling to 10°C, zidovudine crystallized from solution as a white solid.
Yield: 0.25g (76%)

The product was shown to be identical in every respect with an authentic sample of zidovudine.

## Claims

1. A novel compound of formula (A).

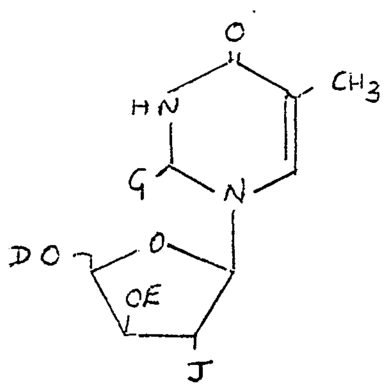

(A)

wherein D represents a methoxycarbonyl group, E represents a mesyl group and J represents a hydrogen atom, hydroxy group, a hydroxy blocking group or a reduceable group and G represents an oxo group or G and J together represent an -0- linkage between the 2 and 2′-positions of the compound of formula (A); or D and E together form a 3′,5-dihydroxy blocking group, J represents a photochemically reduceable group or hydrogen and G is an oxo group.

2. A novel compound of formula (B)

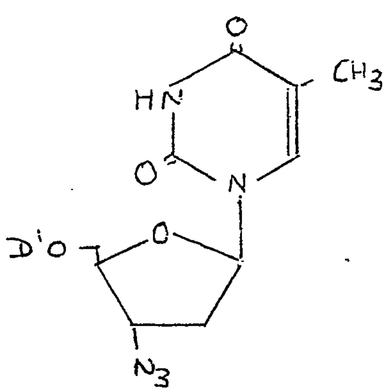

(B)

wherein $D^1$ represents a methoxycarbonyl group, for use in the synthesis of zidovudine.

3. A novel compound of formula (C)

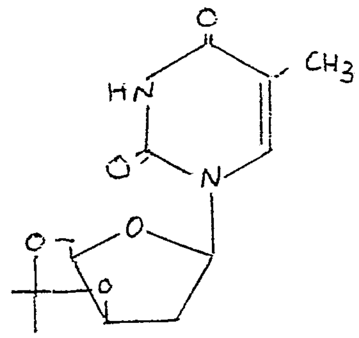

(C)

for use in the synthesis of zidovudine.

4. A novel compound of formula (D)

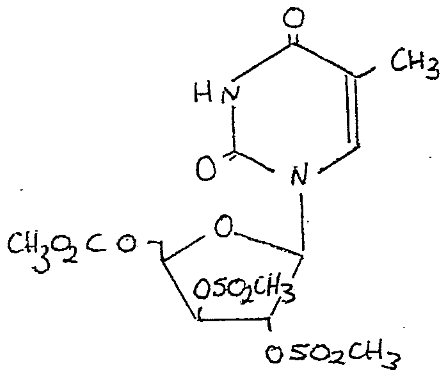

(D)

for use in the synthesis of zidovudine.

5. A novel compound of formula (E)

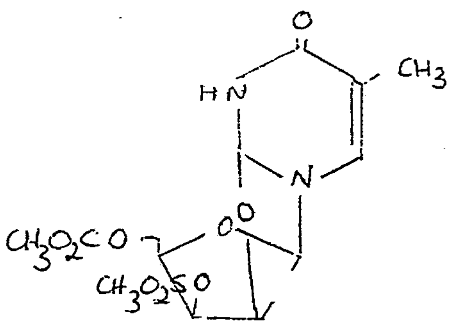

(E)

for use in the synthesis of zidovudine.

6. A novel compound of formula (F)

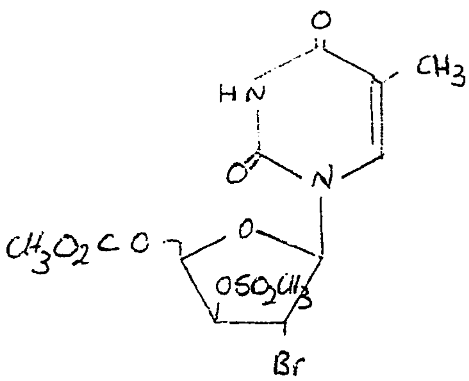

(F)

for use in the synthesis of zidovudine.

7. A novel compound of formula (G)

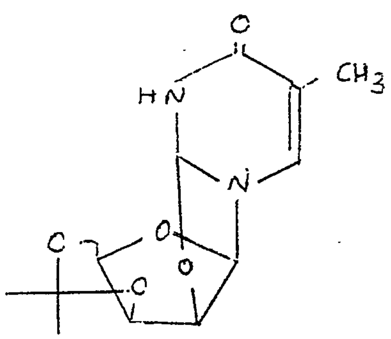

(G)

for use in the synthesis of zidovudine.

8. A novel compound of formula (H)

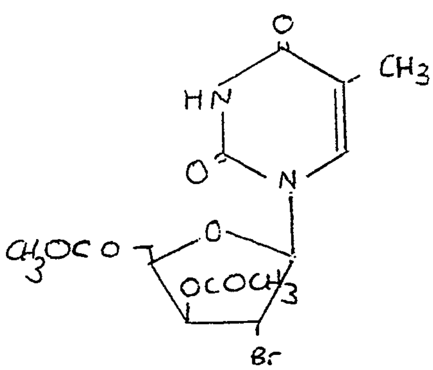

(H)

for use in the synthesis of zidovudine.

9. A novel compound of formula (I)

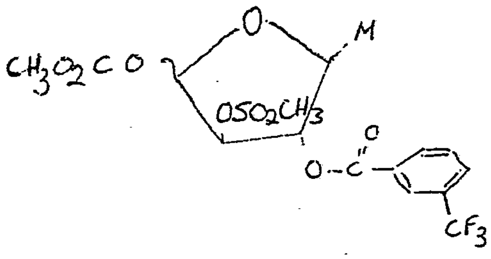

(I)

wherein M is a $CH_3$ or acetyl group, for use in the synthesis of zidovudine.

10. A novel compound of formula (J)

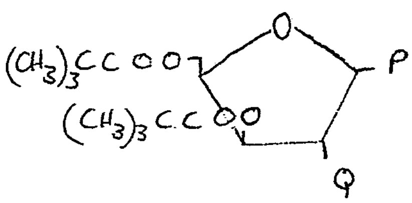

(J)

wherein P and Q are both hydroxy groups or together forma cyclic sulphate group, for use in the synthesis of zidovudine.

11. A process for the preparation of zidovudine which involves the 5′ deblocking of a compound of formula (B)

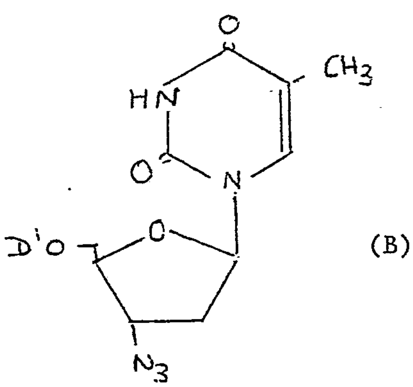

(B)

wherein $D^1$ is methoxycarbonyl.

12. A process for the preparation zidovudine which involves the photochemical deoxygenation of a compound of formula ($A^1$)

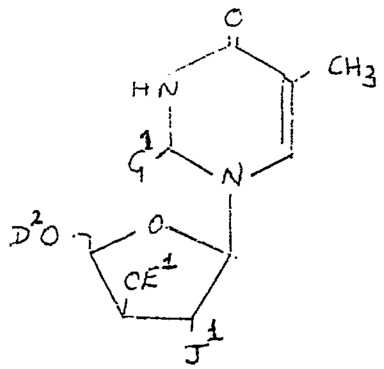

$(A^1)$

wherein $D^2$ represents a methoxycarbonyl group, $E^1$ represents a mesyl group and $J^1$ represents a photochemically reduceable group, $G^1$ represents an oxo group; or $D^2$ and $E^1$ together form a hydroxy blocking group, represents a photochemically reduceable group and $G^1$is an oxo group.

13. A process for the preparation of zidovudine involving:

a) the photolytic reduction of a compound of formula (A) as defined in claim (1),

b) the introduction of an azide group to the 3′-position of the sugar moiety of the resulting product of stage a),

c) the subsequent 5′- deblocking of the resulting product of stage b) compound to give zidovudine.

14. A process for the preparation of zidovudine from D-xylose involving:-

a) treating D-xylose,

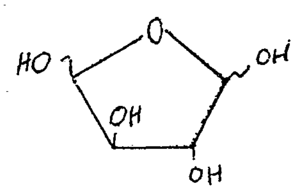

(I)

with a selective hydroxy blocking agent to form a compound of formula (II)

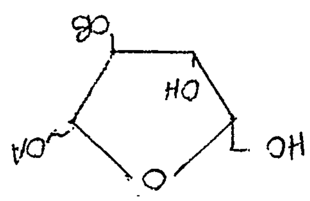

(II)

in which A and B each represent a hydroxy blocking group or together form a single such group serving to block the 1- and 2- hydroxy groups;

b) treating a compound of formula (II) above with further hydroxy blocking agents which either selectively block the 5- and/or 3-hydroxy groups or together form a single group blocking the 5- and 3-hydroxy groups;

i) optionally treating the resulting compound with an agent capable of deblocking the 1- and 2- hydroxy groups and optionally introducing a cyclic sulphite group blocking the 1- and 2-hydroxy groups;

ii) treating the resulting compound with an agent serving to introduce a leaving group at the 3-position of the sugar ring and optionally replacing any combined groups blocking the 1- and 2- hydroxy groups with single such blocking groups or a single group blocking the 1- hydroxy group, the 2- hydroxy group being optionally blocked by a photolytically reduceable group; and then

iii) optionally introducing a leaving group at the 1- hydroxy position of the sugar ring;

c) reacting the resulting compound with a thymine derivative of formula (III)

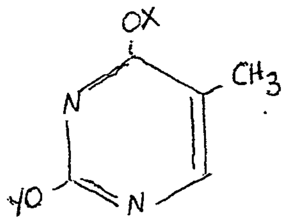

(III)

(wherein X and Y each represent an activating group), to form a compound of formula (IV)

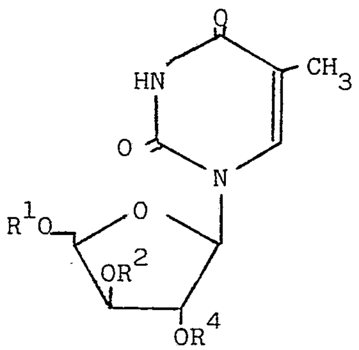

(IV)

(wherein $R^1$ is a hydroxy blocking group, $R^2$ is a leaving group, or $R^1$ and $R^2$ are individual hydroxy blocking groups or together form a single hydroxy blocking group and $R^4$ is hydrogen or a hydroxy blocking group preferably a photolytically reduceable group);

d) i) optionally deblocking the 3′ and 5′- hydroxy groups and reblocking with alternative blocking groups (wherein $R^4$ is a non-photolytically reduceable hydroxy blocking group, treating a compound of formula (IV) with an agent to remove the 2′- hydroxy blocking group), then treating the 2′- hydroxy compound with an agent serving to provide a photolytically or non-photolytically reduceable group at the 2′- position of the sugar moiety directly, or by :

1) introducing a leaving group at the 2′- position of the sugar moiety;

2) cyclising the compound to form a 2′,2- anhydro derivative and either

a) opening the ring to give a compound of formula (V)a or (V)b;

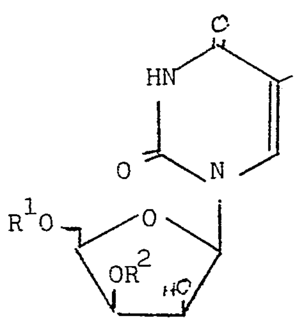

(V)a

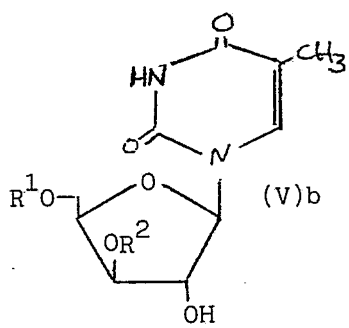

(V)b

(wherein $R^1$ and $R^2$ are as hereinbefore defined) and introducing a photolytically reduceable group at the 2′-position of the sugar ring; or

b) introducing a non-photolytically reduceable group at the 2′-position of the sugar ring, and reducing to form a compound of formula (VI) below;

d) ii) treating a compound of formula (IV) wherein $R^4$ is a photolytically reduceable group or a compound from d) i) above to deoxygenate the 2′- position sugar moiety to form a compound of formula (VI);

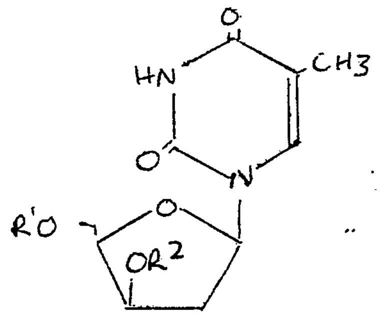

(VI)

(wherein $R^1$ and $R^2$ are as hereinbefore defined)

(e) where a compound of formula (VI) is formed wherein $R^1$ and $R^2$ together form a single hydroxy blocking group, treating the compound with a suitable agent to effect deblocking and selective blocking of the 5′-hydroxy group and introduction of a suitable leaving group at the 3′-position of the 5′-blocked compound;

f) reacting the compound of formula (VI) with an agent serving to introduce an azido group at the 3′-position in the erythro configuration to form a compound of formula (VII)

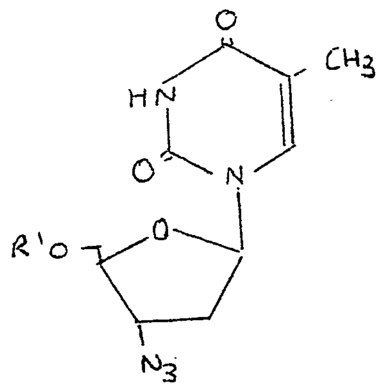

(VII)

(in which $R^1$ is as hereinbefore defined) and

g) removing the 5′-hydroxy protecting group from the compound of formula (VII) to form zidovudine.